Europäisches Patentamt

European Patent Office

Office européen des brevets

(19)

(11) Veröffentlichungsnummer: **0 229 927 B1**

(12) **EUROPÄISCHE PATENTSCHRIFT**

(45) Veröffentlichungstag der Patentschrift: **12.06.91**

(21) Anmeldenummer: 86116374.9

(22) Anmeldetag: **25.11.86**

(51) Int. Cl.⁵: **B09B 3/00**, C05F 9/00, C05F 9/02, C05F 11/04

(54) **Verfahren zur Entsorgung der organischen Hausmüllfraktion.**

(30) Priorität: **21.12.85 DE 3545679**

(43) Veröffentlichungstag der Anmeldung: **29.07.87 Patentblatt 87/31**

(45) Bekanntmachung des Hinweises auf die Patenterteilung: **12.06.91 Patentblatt 91/24**

(84) Benannte Vertragsstaaten: **AT BE CH DE ES FR GB GR IT LI LU NL SE**

(56) Entgegenhaltungen: **DE-A- 3 309 772 JP-A-53 012 165 US-A- 4 012 219**

(73) Patentinhaber: **Preussag Noell Wassertechnik GmbH Alfred-Nobel-Strasse 20 W-8700 Würzburg 1(DE)**

(72) Erfinder: **Wildenauer, Franz Xaver Zehntfeldstrasse 161 W-8000 München82(DE)**

(74) Vertreter: **Kaiser, Henning c/o Preussag AG, Patente und Lizenzen, Postfach 15 12 27 Kurfürstendamm 32 W-1000 Berlin 15(DE)**

Rank Xerox (UK) Business Services

## Beschreibung

Die Erfindung betrifft ein Verfahren zur Entsorgung der organischen, in der Regel Schwermetallsalze enthaltenden Hausmüllfraktion.

Hausmüll enthält durchschnittlich etwa 35 bis 50 % an organischer Substanz, welche sich im Rahmen einer Vorsortierung durch entsprechende Methoden von den übrigen Müllbestandteilen trennen läßt. Ökologisch besonders problematisch ist die in der Regel gegebene Anreicherung der organischen Hausmüllfraktion mit giftigen Schwermetallverbindungen.

Die in der Bundesrepublik Deutschland jährlich anfallende Hausmüllmenge von ca. 15 Mio Tonnen wird bislang größtenteils auf etwa 530 geordneten Deponien abgelagert oder in Müllverbrennungsanlagen pyrolytisch zersetzt. Hinsichtlich der erwähnten Schwermetallverbindungen besteht bei Deponien die Gefahr, daß sie vom Regen ausgewaschen werden und letztlich in das Grundwasser gelangen, beim Verbrennen wiederum besteht die Gefahr, daß die giftigen Verbindungen zur weiteren Luftverschmutzung beitragen. Das Deponieren besitzt darüber hinaus die Nachteile, daß es sehr viel Platz benötigt und daß es keinerlei Energierückgewinnung ermöglicht. Ein kommendes Verfahren, welches bislang nur etwa 3 % der Haushalte entsorgt, ist die Kompostierung. Bei der Kompostierung werden leicht zersetzliche, organische Bestandteile des zu diesem Zweck vorsortierten Hausmülls in einem Rotteprozeß unter Sauerstoffzufuhr bei gleichzeitiger Wärmeentwicklung aufoxidiert, wobei ein hygienisiertes Endprodukt mit deutlich verringertem Volumen entsteht. Insofern ist die Kompostierung eine wirkungsvolle Maßnahme zur Verringerung der Abfallmenge. Allerdings verbleiben bei diesem Verfahren Schadstoffe wie z.B. Schwermetalle im Kompost, was einer weiteren Verwendung, beispielsweise zur Bodenverbesserung in der Landwirtschaft, entgegensteht.

Ausgehend von der Tatsache, daß mehr als 60 % des gesamten Hausmülls aus biologisch abbaubarer Zellulose in Form von Lebensmittelresten, Papier und Pappe besteht, wurde mehrfach versucht, die organische Müllfraktion mit oder ohne Klärschlamm einem anaeroben Abbau zu unterziehen, mit dem Ziel, neben der biologischen Stabilisierung zusätzlich Energie in Form von Methangas zu gewinnen. Allerdings traten bei den üblichen Reaktorbauarten durch die anaerob nicht oder schwer abbaubaren, organischen Substanzen erhebliche Mischprobleme durch Schwimmbecken- bzw. Sedimentbildung auf, was eine wirtschaftliche Arbeitsweise, sprich Biogasgewinnung, unmöglich machte. In diesem Zusammenhang ist es auch bekannt, daß anaerobe Reaktoren geeignet sind, Schwermetalle wie Zc, Cu, Cr, Ni, Pb, Hg und Co bis zu 90 % durch die Bildung unlöslicher Metallsulfide zurückzuhalten.

Aus der DE-A-33 09 772 ist ein Verfahren zum Abtrennen von Schwermetallen aus Schlämmen bekannt, wobei unter dem Begriff "Schlämme" in Kläranlagen anfallende Klärschlämme, aber auch Sedimente, wie beispielsweise in Flüssen, insbesondere hinter Kläreinrichtungen, zu verstehen sind.

Die grundsätzliche Vorgehensweise bei dem dort beschriebenen Verfahren ist die, daß der Schwermetalle enthaltende Schlamm in einer Chloridlösung in einem Laugebehälter einer oxidierenden Laugung unterworfen und während der Laugung bewegt wird, und daß abschließend eine Trennung von Feststoffen und Flüssigkeit durchgeführt wird.

Der weitaus größte Teil der Schwermetalle geht dabei als lösliche Chloride in die Flüssigkeit über und kann anschließend mit einem Fällungsmittel, z.B. einem Alkalisulfid, in Form schwer löslicher Sulfide aus der Lauge entfernt werden.

Die nunmehr schwermetallarmen Feststoffe können unmittelbar landwirtschaftlich verwertet werden, nämlich als Dünger zur Bodenverbesserung.

Die abgezogene Lauge kann durch Zugabe von Salzsäure und Chlorid "nachgeschärft" und erneut zur Laugung verwendet werden. Wie in der deutschen Offenlegungsschrift beschrieben, ist das Verfahren auch in mehreren Stufen durchführbar, d.h. mit wiederholter Laugung und Trennung der festen und flüssigen Bestandteile.

Dieses offensichtlich sehr wirkungsvolle Verfahren der oxidierenden Laugung in einer Chlorid-Lösung ist speziell auf stark schwermetallhaltigen, kompakten Kärschlamm abgestimmt und somit nicht unmittelbar für organischen Hausmüll verwendbar.

Gegenüber den genannten Entsorgungsarten besteht die Aufgabe der Erfindung darin, ein Verfahren zur Entsorgung der durch Vorsortierung ausgesonderten, organischen Hausmüllfraktion anzugeben, welches in einem zusammenhängenden Prozeß eine deutliche Reduzierung des Müllvolumens, ein gezieltes Abscheiden von Schwermetallverbindungen sowie eine wirtschaftlich sinnvolle Energierückgewinnung in Form des im Biogas enthaltenden Energieträgers Methan ermöglicht.

Diese Aufgabe wird durch das im Anspruch 1 wiedergegebene Verfahren gelöst.

Somit kombiniert die Erfindung im wesentlichen drei Methoden, nämlich die Laugung, die Kompostierung (Rotte) und die Methanisierung. Dabei laufen die beiden ersten, aeroben Verfahrensschritte in der Laugungsanlage räumlich und zeitlich gemeinsam ab, wohingegen der anaerobe, dritte Verfahrensschritt in einem getrennten Festbettreaktor im Anschluß an die beiden ersten stattfindet. Durch den aeroben Rotteprozeß wird das Müllvolumen deutlich reduziert, durch die Laugung werden die am besten für die Methanisierung geeigneten Substanzen und die Schwermetallverbindungen ausgewaschen, so daß der aus der Laugungsanlage kommende, im wesentlichen feste, entgiftete Kompost bedenkenlos deponiert oder als landwirtschaftlicher Dünger verwendet werden kann. Das Entfernen dieser festen Substanzen aus dem Prozeßablauf ist eine entscheidende Voraussetzung für einen störungsfreien Betrieb des Festbettreaktors. Der den flüssigen Ablauf vorübergehend aufnehmende Zwischentank dient dazu, die Methanisierung von den teilweise instationären Betriebszuständen in der Laugungsanlage abzukoppeln. Der Festbettreaktor erfüllt zwei Aufgaben, nämlich zum einen die Energierückgewinnung in Form der Biogas- und somit Methanerzeugung, zum anderen das Ausfällen der Schwermetalle in Form wasserlöslicher Sulfide, welche dadurch gezielt entfernt und ggf. weiterverarbeitet werden können. Bei günstiger Auslegung der Anlage ist damit zu rechnen, daß das gewonnene Methangas mehr Energie enthält, als für den Betrieb der Anlage erforderlich ist.

Die Unteransprüche 2 bis 5 enthalten bevorzugte Ausgestaltungen des im Hauptanspruch gekennzeichneten Verfahrens.

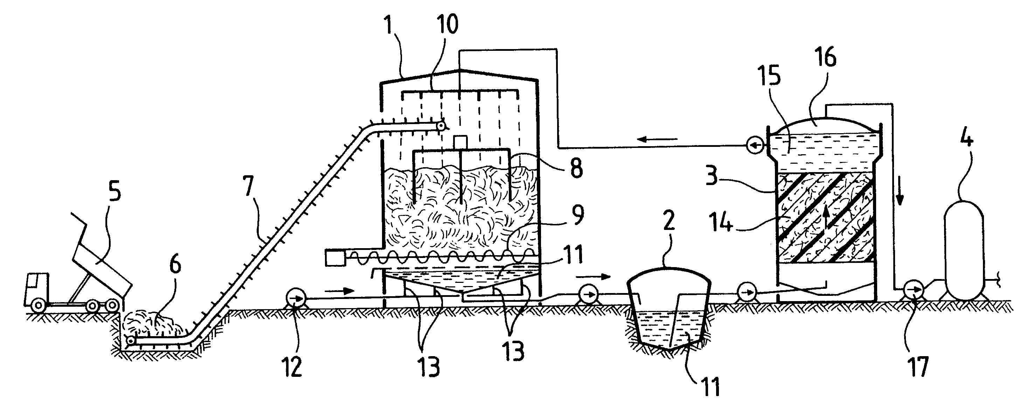

Die Erfindung wird im folgenden anhand des in der Zeichnung dargestellten Ausführungsbeispieles noch näher erläutert. Dabei zeigt die Figur in schematischer Darstellung eine Anlage zur Durchführung des erfindungsgemäßen Entsorgungsverfahrens.

Links in der Figur ist ein Lastkraftwagen 5 zu erkennen, welcher seine aus organischer Hausmüllfraktion 6 bestehende Ladung soeben auf ein Förderorgan 7 gekippt hat. Die Ladung wurde bei einer Vorsortieranlage abgeholt, wo sie von den anorganischen Hausmüllbestandteilen, wie z.B. Metallen, abgesondert und ggf. noch zerkleinert worden war. Die organische Hausmüllfraktion 6 wird von dem Förderorgan 7, welches beispielsweise ein Förderband, ein Kratzkettenförderer oder ein Becherwerk sein kann, von oben in die siloartige Laugungsanlage 1 transportiert und dort von einem beispielsweise rührwerkähnlichen Verteiler 8 gleichmäßig ausgebreitet. Von oben wird die Hausmüllfraktion 6 aus Rieselrohren 10 mit einer Flüssigkeit besprüht, welche hauptsächlich aus Wasser besteht und als Überlauf 15 dem Festbettreaktor 3 entnommen wird. Diese wässrige Flüssigkeit wäscht die wasserlöslichen, organischen Substanzen und die ebenfalls wasserlöslichen Schwermetallsalze aus der Hausmüllfraktion 6 heraus und sammelt sich im Bodenbereich der Laugungsanlage 1. Gleichzeitig mit diesem Laugungsprozeß wird von einem Kompressor 12 über Luftdüsen 13 aus der Umgebung angesaugte Druckluft von unten in die Laugungsanlage 1 eingeblasen, welche zumindest einen Teil der wasserunlöslichen, organischen Substanzen oxidiert, somit hygienisiert und volumenmäßig verkleinert. Die Geschwindigkeit dieses aeroben Kompostierungsprozesses läßt sich über den Volumenstrom der Preßluft beeinflussen. Die weitgehend abgebauten, festen, organischen Bestandteile werden mittels eines Förderorganes 9, hier in Form eines Schneckenförderers, von unten aus der Laugungsanlage 1 heraustransportiert und beispielsweise deponiert oder als Dünger weiterverwendet. Mit diesen festen Substanzen geht dem Laugungskreislauf natürlich auch Flüssigkeit verloren, welche durch Wasserzufuhr wieder ersetzt wird. Der flüssige Ablauf 11, welcher die für die weitere, anaerobe Behandlung geeigneten Substanzen in gelöster Form enthält, wird abgepumpt und im Zwischentank 2 gesammelt. Auf diese Weise kann der Ablauf 11 je nach Bedarf dem Festbettreaktor 3 zugeführt werden. Im Festbett 14 des Reaktors 3, welches von unten nach oben durchströmt wird, bauen anaerobe Bakterien die gelösten organischen Substanzen zu Kohlendioxid und Methan, also zu Biogas ab. Das Biogas sammelt sich unter dem auf der Flüssigkeitsoberfläche schwimmenden, kuppelförmigen Deckel des Festbettreaktors 3 und kann von dort abgesaugt werden. Es wird zweckmäßigerweise über einen Verdichter 17 unter Verringerung des Volumens in einen Niederdruckspeicher 4 gefördert, wo es als wertvoller Energieträger für die weitere Verwertung, beispielsweise für die Verbrennung, zur Verfügung steht.

In der reduzierenden Atmosphäre des Festbettreaktors 3 werden die gelösten Schwermetallsalze zu wasserunlöslichen Sulfiden umgebaut und fallen in fester Form aus der Flüssigkeit aus. Diese Sulfide können aus dem Laugungskreislauf selektiv entfernt und somit einer kontrollierten Weiterverarbeitung oder Deponierung zugeführt werden.

Der flüssige Überlauf 15 des von unten nach oben durchströmten Festbettreaktors wird in der Nähe des Reaktordeckels abgezogen und der Laugungsanlage 1 als Laugungsflüssigkeit zugeführt. Durch diesen geschlossenen Laugungskreislauf ist es möglich, den Wasserverbrauch der Anlage sehr niedrig zu halten, da nur die relativ geringen Flüssigkeitsverluste ausgeglichen werden müssen.

Somit stellt das erfindungsgemäße Verfahren sowohl hinsichtlich der Umweltfreundlichkeit als auch hinsichtlich der Wirtschaftlichkeit eine den bekannten Entsorgungsmethoden deutlich überlegene Lösung dar.

**Ansprüche**

1. Verfahren zur Entsorgung der organischen, in der Regel Schwermetallsalze enthaltenden Hausmüllfraktion mit folgenden Verfahrensschritten:
   - Vorsortieren der organischen Hausmüllfraktion (6) aus gewöhnlichem Hausmüll
   - Einbringen der organischen Hausmüllfraktion (6) in eine Laugungsanlage (1)
   - Gleichmäßiges Verteilen (Verteiler 8) innerhalb der Laugungsanlage (1)
   - Laugung mit einer hauptsächlich aus Wasser bestehenden Flüssigkeit, Herauswaschen der wasserlöslichen organischen Substanzen sowie der wasserlöslichen Schwermetallsalze aus der Hausmüllfraktion (6)
   - Aerobe Umsetzung (Rotte) der organischen Substanzen durch gezielte Belüftung (Luftdüsen 13) während der Laugung
   - Ausfördern (Förderorgan 9) der nicht gelösten Substanzen aus der Laugungsanlage (1) zur weiteren Verarbeitung, beispielsweise zur Deponierung
   - Abziehen des die gelösten Substanzen enthaltenden, flüssigen Ablaufes (11) aus der Laugungsanlage (1)
   - Sammeln des Ablaufes (11) in einem Zwischentank (2)
   - Abziehen des Ablaufes (11) aus dem Zwischentank (2) nach Bedarf
   - Einbringen des Ablaufes (11) in einen anaeroben Festbettreaktor (3)
   - Abbau der gelösten organischen Substanzen zu Methan und Kohlendioxid (Biogas 16) durch anaerobe Bakterien im Festbett (14) des Reaktors (3)
   - Ausfällen der Schwermetallverbindungen durch Umsetzung der gelösten Schwermetallsalze in wasserunlösliche Schwermetallsulfide
   - Rückführung des flüssigen Überlaufes (15) vom Festbettreaktor (3) zur Laugungsanlage (1) zum Bewässern der Hausmüllfraktion (6) (Laugungskreislauf)
   - Abziehen des Biogases (16) aus dem Festbettreaktor (3) zur weiteren Verwendung, beispielsweise als Brennstoff.

2. Verfahren nach Anspruch 1, **dadurch gekennzeichnet**, daß die Laugung bzw. die aerobe Umsetzung der Hausmüllfraktion (6) in einer weitgehend geschlossenen, siloartigen Laugungsanlage (1) abläuft, daß das Einbringen in die Laugungsanlage (1) von oben erfolgt, daß die Laugung der Hausmüllfraktion (6) durch Berieseln (Rieselrohre 10) von oben erfolgt, daß für die aerobe Umsetzung zusätzlich zu der in der Laugungsanlage (1) vorhandenen Umgebungsluft von unten Preßluft in die Hausmüllfraktion eingeblasen wird (Luftdüsen 13), daß das Ausfördern der nicht gelösten Substanzen schichtweise von unten her erfolgt, und daß der flüssige Ablauf (11) an der tiefsten Stelle der Laugungsanlage (1) gesammelt und von dort abgezogen wird.

3. Verfahren nach Anspruch 1 oder 2, dadurch **gekennzeichnet**, daß die im wesentlichen auf das Ausfördern mit den nicht gelösten Substanzen aus der Laugungsanlage zurückzuführenden Wasserverluste des Laugungskreislaufes ständig oder in zeitlichen Abständen ergänzt werden.

4. Verfahren nach einem oder mehreren der Ansprüche 1 bis 3, dadurch **gekennzeichnet**, daß die ausgefällten Schwermetallsulfide bei Erreichen einer gewissen Menge bzw. Konzentration aus dem Laugungskreislauf entfernt werden.

5. Verfahren nach einem oder mehreren der Ansprüche 1 bis 4, dadurch **gekennzeichnet**, daß das im Festbettreaktor (3) erzeugte Biogas (16) in einem getrennten Niederdruckspeicher (4) gesammelt wird.

**Claims**

1. Method of removing the organic portion of household refuse, generally containing heavy-metal salts, using the following method steps:
   - pre-sorting the organic portion of household refuse (6) from the usual household refuse;
   - introducing the organic portion of household refuse (6) into a leaching plant (1);
   - effecting uniform distribution (distributor 8) internally of the leaching plant (1);
   - leaching with a fluid, mainly comprising water, washing-out the water-soluble, organic substances and the water-soluble heavy-metal salts from the portion of household refuse (6);
   - aerobically converting (rotting) the organic substances by appropriate aeration (air nozzles 13) during the leaching process;
   - conveying (conveyor means 9) the non-dissolved substances from the leaching plant (1) for further processing, for example for storage;
   - drawing-off the liquid effluent (11), which contains the dissolved substances, from the leaching plant (1);

- collecting the effluent (11) in an intermediate tank (2);
- drawing-off the effluent (11) from the intermediate tank (2), as required;
- introducing the effluent (11) into an anaerobic fixed-bed reactor (3);
- decomposing the dissolved, organic substances to form methane and carbon dioxide (biogas 16) by means of anaerobic bacteria in the fixed bed (14) of the reactor (3);
- precipitating the heavy-metal compounds by converting the dissolved heavy-metal salts into water-insoluble heavy-metal sulphides;
- returning the liquid overflow (15) from the fixed-bed reactor (3) to the leaching plant (1) in order to water the portion of domestic refuse (6) (leaching cycle); and
- drawing-off the biogas (16) from the fixed-bed reactor (3) for further use, for example as a fuel.

2. Method according to claim 1, characterised in that the leaching, or respectively the aerobic conversion, of the portion of household refuse (6) is effected in a largely sealed, silo-like leaching plant (1), in that the introduction of the refuse into the leaching plant (1) is effected from above, in that the leaching of the portion of household refuse (6) is effected by trickling (trickle pipes 10) from above, in that, for the aerobic conversion, in addition to the ambient air present in the leaching plant (1), compressed air is injected into the portion of household refuse from below (air nozzles 13), in that the non-dissolved substances are conveyed-out from below in layers, and in that the liquid effluent (11) is collected at the lowest point of the leaching plant (1) and is drawn-off from there.

3. Method according to claim 1 or 2, characterised in that the water losses in the leaching cycle, which are substantially caused by conveying the non-dissolved substances from the leaching plant, are restored constantly or at timely intervals.

4. Method according to one or more of claims 1 to 3, characterised in that the precipitated heavy-metal sulphides are removed from the leaching cycle upon the attainment of a predetermined quantity or concentration.

5. Method according to one or more of claims 1 to 4, characterised in that the biogas (16), which is produced in the fixed-bed reactor (3), is collected in a separate low-pressure storage tank.

## Revendications

1. Procédé de séparation et de traitement de la partie organique d'ordures ménagères contenant en règle générale des sels de métaux lourds, procédé qui comporte les étapes suivantes consistant à:
    - procéder à un tri préalable pour séparer d'ordures ménagères courantes la partie organique d'ordures ménagères (6)
    - admettre la partie organique d'ordures ménagères (6) dans une installation de lixiviation (1)
    - procéder à une répartition uniforme (répartiteur 8) à l'intérieur de l'installation de lixiviation (1)
    - procéder à une lixiviation à l'aide d'un liquide constitué principalement d'eau, extraire par lavage de la partie d'ordures ménagères (6) les substances organiques solubles dans l'eau ainsi que les sels de métaux lourds solubles dans l'eau
    - procéder à une transformation aérobie (rouissage) des substances organiques par une aération appropriée (buses d'air 13) pendant la lixiviation
    - évacuer (organe transporteur 9) de l'installation de lixiviation (1) les substances non dissoutes en vue de leur traitement ultérieur, par exemple, en vue de leur décharge
    - extraire de l'installation de lixiviation (1) le liquide d'écoulement (11) contenant les substances dissoutes
    - collecter le liquide d'écoulement (11) dans un réservoir intermédiaire (2)
    - extraire du réservoir intermédiaire (2) le liquide d'écoulement (11), en fonction des besoins
    - admettre le liquide d'écoulement (11) dans un réacteur à lit fixe anaérobie (3)
    - procéder à une décomposition des substances organiques dissoutes en méthane et Gaz carbonique (biogaz 16) par des bactéries anaérobies dans le lit fixe (14) du réacteur (3)
    - précipiter les composés de métaux lourds par transformation des sels de métaux lourds dissous en sulfures de métaux lourds non solubles dans l'eau
    - recycler dans l'installation de lixiviation (1), le tropplein liquide (15) du réacteur à lit fixe (3), pour arroser la partie d'ordures ménagères (6) (cycle de lixiviation)
- extraire du réacteur à lit fixe le biogaz (16) en vue de son utilisation ultérieure, par exemple, comme combustible.

2. Procédé selon la revendication 1, caractérisé en ce que la lixiviation, ou la transformation aérobie, de la partie d'ordures ménagères (6) se déroule dans une installation de lixiviation semblable à un silo, largement fermée, en ce que l'admission dans l'installation de lixiviation (1) se fait depuis le haut, en ce que la lixiviation de la partie d'ordures ménagères (6) se fait par ruissellement (tuyau de ruissellement 10) depuis le haut, en ce que, pour la transformation aérobie, de l'air sous pression est insufflé depuis le bas (buses d'air 13) dans la partie d'ordures ménagères, en plus de l'air ambiant présent dans l'installation de lixiviation (1), en ce que l'évacuation des substances non dissoutes se fait par couches à partir du bas, et en ce que le liquide d'écoulement (11) est collecté au point le plus bas de l'installation de lixiviation (1) et extrait à partir de ce point.

3. Procédé selon la revendication 1 ou 2, caractérisé en ce que les pertes d'eau du cycle de lixiviation, qui sont dues pour l'essentiel à l'évacuation d'eau conjointement avec les substances non dissoutes hors de l'installation de lixiviation, sont compensées d'une manière constante ou suivant des intervalles de temps.

4. Procédé selon l'une au moins des revendications 1 à 3, caractérisé en ce que les sulfures de métaux lourds précipités sont éliminés du cycle de lixiviation, lorsqu'ils atteignent une certaine quantité ou concentration.

5. Procédé selon l'une au moins des revendications 1 à 4, caractérisé en ce que le biogaz (16) produit dans le réacteur à lit fixe (3) est collecté dans un réservoir à basse pression séparé (4).

EP 0 229 927 B1